Europäisches Patentamt

European Patent Office

Office européen des brevets

(19)

(11) EP 0 951 905 A2

(12) EUROPEAN PATENT APPLICATION

(43) Date of publication:
27.10.1999 Bulletin 1999/43

(51) Int Cl.⁶: **A61K 31/275, A61K 9/32**

(21) Application number: 98201189.2

(22) Date of filing: 14.04.1998

(84) Designated Contracting States:
**AT BE CH CY DE DK ES FI FR GB GR IE IT LI LU MC NL PT SE**
Designated Extension States:
**AL LT LV MK RO SI**

(30) Priority: 24.04.1997 IT MI970959

(71) Applicant: **APR APPLIED PHARMA RESEARCH S.A.**
**6855 Stabio (CH)**

(72) Inventors:
• **Maggi, Lauretta**
**27100 Pavia (IT)**
• **Conte, Ubaldo**
**27100 Pavia (IT)**
• **Reiner, Alberto**
**22100 Como (IT)**

(74) Representative: **Dragotti, Gianfranco et al**
**DRAGOTTI & ASSOCIATI SRL,**
**Galleria San Babila, 4/C**
**20122 Milano (IT)**

(54) **Pharmaceutical compositions for programmed therapeutical coverage**

(57) New pharmaceutical compounds for oral use that contain Verapamil hydrochlorate as an active ingredient are described; these compounds are made up of one or more completely coated tablets or granules.

The formulations according to this invention make it possible to ensure the delayed and programmed release of the active ingredient and a high level of bioavailability thereof.

**Description**

This invention pertains to new pharmaceutical compounds for oral use that contain Verapamil hydrochlorate as an active ingredient and that make it possible to ensure the delayed and programmed release of the active ingredient.

In recent decades significant advances have been made in producing so-called "retard" pharmaceutical formulations that are thus able to release the active ingredient contained therein at a rate that is appropriately controlled and delayed.

In order to obtain extended "therapeutic coverage", that is, to ensure that therapeutically effective plasma levels of the active substance are maintained and thus to be able to reduce the number of times it is administered daily, numerous prototypes of pharmaceutical formulations adapted to this purpose have been studied and produced.

One of the basic requirements for all pharmaceutical formulations that are referred to as "retard" formulations is that they be able to contain elevated quantities of active ingredient which, once in the organism, is released at predetermined times, thereby ensuring that it remains pharmacologically active for an extended length of time.

The regular release of the active ingredient observed in the in vitro release trials is not always reproduced, however, when the pharmaceutical formulation is administered to human subjects; as a matter of fact, it has been found that, in many cases, the absorption of the active ingredient in the organism is inadequate, thus leading to suboptimum plasma levels and therefore limiting the corresponding therapeutic effect.

A typical case of this problem, which is basically associated with the irregular or at least inadequate absorption of the active ingredient, is that of 5-[(3,4-dimethoxyphenylethyl)-methylamine]-2-(3,4 dimethoxyphenyl)-2-isopropylvaleronitrile, more commonly known as Verapamil hydrochlorate.

This medication, which is well known for its hypotensive and anti-arrhythmic properties, is marked by the fact that it reaches plasma levels that are surprisingly far below those that would be expected based on the most common in vitro experimental models; the therapeutic action of Verapamil hydrochlorate is thus significantly compromised, and the use of especially high doses, which leads, of course, to correspondingly high production costs, rarely translates into improved results.

The formulation of the Verapamil hydrochlorate-containing pharmaceutical compounds that are known to the state of the art is based on the now universally acknowledged and accepted principle that the absorption of the active ingredient in question should begin in the stomach and then continue progressively in the small intestine and, finally, in the large intestine.

The "retard" pharmaceutical compounds based on Verapamil hydrochlorate that are currently on the market, for example ISOPTIN (produced by Knoll Pharmaceuticals), are, in fact, manufactured in such a way as to make it possible to release the active ingredient under the typical acidity conditions that prevail in the stomach (pH ≤ 4).

It has now been found, surprisingly enough, that, contrary to what is stated above, the problem of this irregular absorption of Verapamil hydrochlorate is largely resolved by properly specifying the part of the digestive system in which the absorption of the active ingredient should begin. In particular, as the examples below will show, it has been found, surprisingly enough, that the absorption of Verapamil hydrochlorate should, in reality, not begin in the stomach but rather should take place only at the level of the large and small intestines.

The object of this invention is therefore a new pharmaceutical compound for oral use with programmed and delayed release of Verapamil hydrochlorate in which the release of the active ingredient begins only after this compound has left the stomach and is thus in the duodenal-intestinal tract of the digestive system.

The pharmaceutical compound that is the object of this invention is composed of one or more tablets (or granules) in which a delayed-release Verapamil hydrochlorate core, which is prepared according to procedures that are conventional and are thus well-known to one skilled in the art, is coated with an appropriate gastro-resistant and entero-soluble film that is thus able to keep the formulation itself intact as it passes through the stomach.

Verapamil hydrochlorate generally comprises 10-90 percent (as Verapamil hydrochlorate) by weight of the core, even though generally the preferred quantity is between 40 and 70 percent.

The gastro-resistant and entero-soluble film generally accounts for 2-15 percent of the weight of the compound, preferably 8-11 percent, and is essentially made of biocompatible and biodegradable polymer materials that are soluble at pH ≥ 5.5, for example cellulose acetophthalate, cellulose acetopropionate, cellulose trimellitate, the co-polymer methylvinylether-maleic anhydride, dibutyl phthalate, and, especially, acrylic and methacrylic co-polymers. These co-polymers can be used either individually or, optionally, in a mixture.

Among the possible acrylic and methacrylic polymers and co-polymers, the ones that are preferred for the implementation of this invention are the co-polymers of the Eudragit(R) series produced by Röhm Pharma.

Using these co-polymers as coating agents for the purposes of carrying active ingredients into the intestine is something that is well known in the pharmaceutical industry; English patent No. GB 2123695 describes delayed-release pharmaceutical formulations of 5-amino-salicylic acid for treating infections of the colon and the rectum that are appropriately coated with Eudragit(R) co-polymers.

Said polymer materials can be used in combination with other retardant polymers or with the addition of other coadjuvants such as dyes, opacifiers, or sweeteners.

It is especially preferred for said film to be supplemented with at least one plasticizer, such as diethyl phthalate, diacetine, triacetine, dibutylphthalate, dibutyltartrate, tributylacetate, castor oil, polyoxyethyleneglycols of different molecular weights, preferably between 200 and 20,000 Dalton, as is well known to one skilled in the art. Said plasticizers may be present at a level of 2-20 percent with respect to the polymer coating material, and preferably 5-15 percent.

The compound according to this invention should also be of such a shape and size that it is not held up in the stomach, but rather is ingested after a meal. In a preferred embodiment of the invention, the formulation comes, for this specific purpose, in the shape of a cylindrical tablet (convex tablet) that has a diameter of between 2 and 12 mm, preferably between 4 and 10 mm, and a height of between 2 and 8 mm, and preferably between 3 and 6 mm.

As mentioned before, with the formulations according to this invention "in vitro" absorption kinetics are obtained that are characterized by reduced variability and elevated bioavailability. This unexpected result is reflected by the detection of the plasma levels that are achieved after a tablet according to this invention is administered to healthy volunteers after a standardized meal.

The pharmaceutical formulations according to this invention may also be placed in a hard-gelatin capsule in order to deliver the desired amount of active ingredient; said capsule may thus contain one or more coated tablets or appropriate amounts of granules, also appropriately coated. Alternatively, said formulations may be contained in an appropriate package that is able to dispense the desired number of tablets in order to provide the necessary individual quantity of the active ingredient.

In the preparation of said tablets (or granules) that are then to be subjected to the phases of coating with the above-described gastro-resistant and entero-soluble film, excipients may be employed that are normally used in the pharmaceutical industry, for example, mannitol, lactose, sorbitol, xylitol, talc, magnesium stearate, colloidal silicas, and others such as glyceryl monostearate, hydrogenated castor oil, waxes, and monosubstituted, bisubstituted, and trisubstituted glycerides.

Since Verapamil hydrochlorate is a highly soluble active ingredient, depending on the amount of active ingredient that is carried at the desired rate of release, it is possible, in the preparation of these compounds, to use excipients that are able to delay the penetration of water and/or aqueous fluids into the formulation itself and thus to slow the rate at which the Verapamil hydrochlorate is released. These excipients may include retardant polymer substances such as, for example, polyvinylpyrrolidone, carboxymethyl sodium-cellulose, carboxymethylamide, potassium methacrylate-divinylbenzene copolymer, polyvinylalcohols, amides, amide derivatives, beta cyclodestrine and destrine derivatives in general, hydroxypropylmethylcellulose with a molecular weight of between 1000 and 4,000,000, hydroxypropylcellulose with a molecular weight of between 2000 and 2,000,000, carboxyvinylpolymers, polyvinylalcohols, glucanes, sclerogulcanes, mannanes, xanthanes, carragenanes, alginic acid and its derivatives, polyanhydrides, polyamino acids, poly-(methyl vinyl ether/maleic anhydride) carboxymethylcellulose and its derivatives, ethylcellulose, methylcellulose, and cellulose derivatives in general. These polymer substances preferably make up 1 to 90 percent by weight of the tablet or the core.

Offered on the market are various types of polymers that fall into the above-mentioned categories and are characterized by different physico-chemical, solubility, and gelification properties; in particular, as regards hydroxypropylmethylcellulose, various kinds of polymers may be used with different molecular weights (from 1,000 to 4,000,000) and with various degrees of substitution. These kinds of hydroxypropylmethylcellulose exhibit differentiated characteristics but are predominantly erodible or predominantly gelifiable, depending on the viscosity and degree of substitution of the polymer.

If, however, it is desired to promote the penetration of water and/or aqueous fluids into the tablet, it is possible to introduce, in particular, hydrophilic diluents such as mannitol, lactose, amides of various kinds, sorbitol, xylitol, and/or hydrophilic excipients in general that promote the penetration of water into the tablet.

The coadjuvants are selected from the group that includes hydrogenated castor oil, glycerylpalmitostearate, cetyl alcohol, polyvinylpyrrolidone, glycerine, ethylcellulose, methylcellulose, sodium carboxymethylcellulose, magnesium stearate, stearic acid, talc, sodium benzoate, boric acid, polyoxyethyleneglycol, and colloidal silicas, and other natural or man-made substances that are well known to one skilled in the art.

In addition, it is also possible to use diluents, bonding agents, lubricants, buffering agents, non-adhering agents, sliding agents, and other substances that are able to impart to this formulation the characteristics that it needs to undergo the process of compression, as will be explained in greater detail in the following examples, which should be regarded solely as illustrative and not as restrictive of the invention.

**Example 1: Preparation of a series of 100,000 tablets that contain 60 mg of Verapamil hydrochlorate as an active ingredient**

1-a-Preparation of the granulate that contains the active ingredient

A granulate is prepared according to the above-described procedures and is used in the preparation of tablets that contain 60 mg of Verapamil hydrochlorate and that have the following unit composition:

- Verapamil hydrochlorate        60.0 mg
- mannitol (C. Erba)        22.0 mg
- hydroxypropylmethylcellulose (Methocel K 15 M)        15.0 mg
- polyvinylpyrrolidone (ethanolic sol, 20 %, GAF, Wayne)        5.0 mg
- talc        3.0 mg
- magnesium stearate        1.5 mg
- colloidal silica        0.5 mg
- Total        107.0 mg

The fabrication process consists of preparing a granulate that is obtained by mixing, in a sigma mixer mod. Erweka type K 5 (Frankfurt a. M., Germany), the appropriate quantities of Verapamil hydrochlorate, mannitol, and hydroxypropylmethylcellulose; the homogeneous powder mixture is bathed with a 20% ethanolic solution of polyvinylpyrrolidone (p/v); the uniformly wet compound is forced through a grid with mesh 25 (710 microns), thus producing a regular granulate that is dried in a fluidized-bed device (Acromatic mod; Strea) with an air inlet temperature of 40-45 degrees Celsius. The granulate, which is dried but has a constant weight, is re-calibrated on a sieve of mesh 25 (710 microns) and is placed in a powder mixer with the addition of magnesium stearate, talc, and colloidal silica.

Mixing proceeds for 20 minutes, and the lubricated granulate is analyzed to determine its content of active ingredient and is then subjected to the phases of compression. A rotary compressor that is equipped with convex circular punches with a diameter of 7.0 mm and a radius of curvature of R=10 mm is used.

The device is adjusted in such a way as to produce tablets with a mean weight of 107 mg, each containing 60 mg of Verapamil hydrochlorate.

The dissolution test as indicated in section 1d below was carried out on a sample of uncoated tablets.

1-b-Coating process

The tablets that are produced as described in 1-a above are subjected to a coating process, whereby a film having the following composition is applied to each of said tablets:

- acrylic and methacrylic acid copolymer (Eudragit L 30 D)        9.34 mg
- polyoxyethyleneglycol        1.66 mg
- Total        11 mg.

The coating process is carried out using a quick coating basin (Manesty Accela-Cota) into which the tablets are poured and in which the tablets, which are continuously rotated, are sprayed, by means of an "air-less" spray system, with a 30 percent aqueous dispersion of the acrylic and methacrylic acid copolymer (Eudragit L 30 D) in which polyoxyethylene glycol has been solubilized. An air inlet temperature of approximately 40-50 degrees is used, according to the technique known to one skilled in the art, thus yielding tablets that are completely coated with a uniform film of the above-mentioned polymer material.

A portion of these coated tablets were used to perform a clinical study of bioavailability.

1-c-Dissolution test (uncoated tablets)

In order to evaluate the characteristics with respect to release of the active ingredient from the prepared (uncoated) tablets, device 2 is used, paddle (USP XXII) operating at 100 rpm and using one liter of a buffer solution at pH 6.8 as the dissolution fluid. The release of the active ingredient is tracked by UV spectrophotometry using an automatic sampling and reading system (Spectracomp - Advanced Products - Milan).

The results of the tests that were performed are presented in Table I.

Table I

| (Uncoated Tablets) | |
|---|---|
| Time (h) | % Released |
| 1 | 7.0 |
| 2 | 20.0 |
| 4 | 44.0 |
| 6 | 70.0 |
| 8 | 92.0 |
| 10 | 100.0 |

1-d-Dissolution test (coated tablets)

In order to evaluate the characteristics with respect to release of the active ingredient from the coated tablets, device 2 is used, paddle (USP XXII) operating at 100 rpm and using one liter of HCl 0.1N as a dissolution fluid for two hours and then replacing the fluid with a buffer solution at pH 6.8. The release of the active ingredient is tracked by UV spectrophotometry using an automatic sampling and reading system (Spectracomp - Advanced Products - Milan).

The results of the tests that were performed are presented in Table II.

Table II

| (Coated Tablets) | |
|---|---|
| Time (h) | % Released |
| 1 | 0.0 |
| 2 | 0.0 |
| 3 | 12.0 |
| 4 | 28.0 |
| 6 | 53.0 |
| 8 | 70.0 |
| 10 | 92.0 |
| 12 | 98.5. |

1-e-In vivo study and verification of bioavailability

In order to confirm whether the new pharmaceutical formulation ensures good bioavailability of the Verapamil hydrochlorate, a single-dose study was carried out on 8 informed, healthy volunteers (4 men and 4 women), whereby the coated pharmaceutical formulation described in section 1d was administered. In particular, the intention was to evaluate whether it is possible to achieve a Verapamil hydrochlorate release after a latency period of 4-6 hours from the time when it is administered after the evening meal and before bed in order to prevent the angina conditions and cardiac arrhythmias that are commonly observed during the early morning hours. For this reason, the experimental protocol called for the volunteers to take, after a standard supper, a single dose of 120 mg of Verapamil hydrochlorate (a hard-gelatin capsule containing two 60 mg coated tablets each). Blood samples were taken from each of the volunteers up until 2200 hours after administration. In these samples the plasma concentrations both of Verapamil hydrochlorate and its metabolite N-desmethylverapamil were determined using a validated chromatographic method.

Table III presents the mean values of the plasma levels of Verapamil hydrochlorate and its metabolite N-desmethylverapamil at various sampling times.

Table III

| Time (h) | Verapamil | N-desmethylverapamil |
|---|---|---|
| 0 | 0.00 | 0.00 |
| 2 | 0.00 | 0.00 |
| 3 | 0.00 | 0.00 |
| 4 | 0.00 | 0.00 |

Table III   (continued)

| Time (h) | Verapamil | N-desmethylverapamil |
|----------|-----------|----------------------|
| 5 | 0.55 | 0.53 |
| 6 | 1.06 | 1.46 |
| 7 | 3.03 | 4.41 |
| 8 | 4.70 | 6.48 |
| 9 | 7.32 | 10.74 |
| 10 | 14.82 | 17.22 |
| 11 | 23.62 | 25.23 |
| 12 | 42.57 | 41.44 |
| 14 | 41.14 | 44.62 |
| 16.5 | 24.49 | 34.02 |
| 18 | 19.00 | 29.68 |
| 20 | 15.40 | 26.71 |
| 22 | 11.64 | 22.28 |

The experimental data that were obtained after healthy volunteers were treated with the above-mentioned formulation were compared to those of two different formulations that had been previously evaluated and that dissolve as early as at the gastric level, i.e., more specifically:

- ISOPTIN 80 mg tablets (equal to a content of 80 milligrams of Verapamil hydrochlorate), whereby the data were normalized to 120 mg;
- 120 mg tablets of Verapamil hydrochlorate in a delayed release formula (RETARD formulations).

The experimental results revealed that:

- the plasma concentrations of Verapamil and N-desmethylverapamil after administration of the formulation that is the subject of this application are still high at the last experimental time (T=22h).

As a matter of fact, a Verapamil value of 11.07 ng/ml was found compared to a value of 0 for the ISOPTIN formulation and 2.6 ng/ml for the RETARD formulation, and a N-desmethylverapamil value of 22.27 ng/ml was found after administration of the formulation that is the subject of this application, compared to 6.72 and 8.12 ng/ml for the other two.

An extrapolation of the AUC was also performed. In this extrapolation, working on the mean concentrations, a half-life of four hours for Verapamil and eight hours for N-desmethylverapamil was assumed.

Since the concentrations of Verapamil and N-desmethylverapamil do not follow the same plots, a more careful comparison was made between the formulations, whereby both the AUC and the $C_{max}$ values of Verapamil and N-desmethylverapamil of the formulation that is the subject of this application and of the comparison formulations were evaluated.

In addition, an evaluation was also made of the $t_{max}$, which turned out to be much more delayed with the formulation that is the subject of this application compared to the comparison formulations, even though this parameter is not especially significant.

The data that were considered are presented in Table IV.

Table IV

| | Formulation | $AUC_{0-t}$ | $AUC_{0-\infty}$ | $C_{max}$ | $t_{max}$ |
|--|-------------|-------------|------------------|-----------|-----------|
| Verapamil | Example 1 | 342 | 411 | 51 | 11.75 |
| | ISOTPIN | 316 | 338 | 67 | 1.67 |
| | RETARD | 260 | 308 | 36 | 3.00 |
| N-desmethylverapamil | Example 1 | 426 | 680 | 49 | 12.37 |
| | ISOTPIN | 571 | 657 | 59 | 3.00 |
| | RETARD | 444 | 578 | 39 | 4.33 |

Table IV   (continued)

| | Formulation | $AUC_{0-t}$ | $AUC_{0-\infty}$ | $C_{max}$ | $t_{max}$ |
|---|---|---|---|---|---|
| Verapamil + N-desmethylverapamil | Example 1 | 762 | 1091 | 100 | - |
| | ISOTPIN | 887 | 995 | 126 | - |
| | RETARD | 704 | 886 | 75 | - |

Contrary to expectations, it was found that, compared to the comparison formulations, the formulations that is the subject of this invention yielded the following results in terms of $AUC_{0-\infty}$ as the sum of Verapamil + N-desmethylverapamil:

$$\text{vs. ISOPTIN } (1091/995) \times 100 = 109.65$$

$$\text{vs. RETARD } (1091/886) \times 100 = 123.14 \text{ percent.}$$

In other words, in terms of the prolongation of the absorption of the formulation that is the subject of this application, bioavailability turned out to be 9.7 percent higher than with ISOPTIN and 23.1 percent higher than with the RETARD formulation.

These results are especially surprising since, in addition to being able to cause a delay in the release of the medication, the formulation that is the subject of this application also ensures a higher and, in particular, a reproducible bioavailability compared to the pharmaceutical formulations that are currently on the market, and this thus represents another innovation with respect to the optimum use of Verapamil.

## Claims

1. Pharmaceutical compound for oral use with delayed and programmed release of Verapamil hydrochlorate, wherein said active ingredient is released only after said compound has reached the duodenal-intestinal tract of the digestive system.

2. Pharmaceutical compound according to claim 1, wherein said compound is composed of one or more tablets or granules in which a delayed-release core of the active ingredient is completely coated with a gastro-resistant and entero-soluble film.

3. Pharmaceutical compound according to claim 2, wherein said film constitutes 2-15 percent of the weight of the compound itself, and preferably 8-11 percent.

4. Pharmaceutical compound according to claim 2, wherein said film is composed essentially of biocompatible and biodegradable polymer materials that are soluble at $pH \geq 5.5$.

5. Pharmaceutical compound according to claim 4, wherein said polymer materials are selected from among cellulose acetophthalate, cellulose acetopropionate, cellulose trimellitate, the copolymer methylvinylether-maleic anhydride, dibutyl phthalate, acrylic and methacrylic polymers and copolymers, and mixtures thereof.

6. Pharmaceutical compound according to claim 2, wherein said film is supplemented with at least one plasticizer.

7. Pharmaceutical compound according to claim 6, wherein said at least one plasticizer is selected from among diethyl phthalate, diacetine, triacetine, dibutylphthalate, dibutyltartrate, tributylacetate, castor oil, and polyoxyethyleneglycols having a molecular weight of between 200 and 20,000 Dalton.

8. Pharmaceutical compound according to claim 7, wherein said at least one plasticizer is present in a quantity of between 2 and 20 percent by weight with respect to the polymer material of the coating, and preferably between 5 and 15 percent.

9. Pharmaceutical compound according to claim 1, wherein it comes in the form of tablets or granules.

10. Pharmaceutical compound according to claim 9, wherein said tablets are cylindrical in shape with a diameter of between 2 and 13 mm and a height of between 2 and 10 mm.

11. Pharmaceutical compound according to claim 9, wherein said tablets are cylindrical in shape with a diameter of between 4 and 8 mm and a height of between 3 and 6 mm.

12. Pharmaceutical compound according to claim 1, wherein Verapamil hydrochlorate constitutes 10-90 percent of the weight of said core.

13. Pharmaceutical compound according to claim 12, wherein Verapamil hydrochlorate constitutes 40-70 percent of the weight of said core.

14. Pharmaceutical compound according to claim 9, wherein said coated tablets or granules are contained in a hard-gelatin capsule.